# EUROPÄISCHE PATENTSCHRIFT

(11) **EP 3 244 848 B1**
(45) Veröffentlichungstag und Bekanntmachung des Hinweises auf die Patenterteilung: **07.06.2023**
(21) Anmeldenummer: 16708071.2
(22) Anmeldetag: 11.01.2016
(51) Int. Cl.: A61F 5/56

(54) **ZWEITEILIGE UNTERKIEFERPROTRUSIONSSCHIENE**
TWO-PART MANDIBULAR ADVANCEMENT SPLINT
GOUTTIÈRE DE PROPULSION MANDIBULAIRE EN DEUX PARTIES

(30) Priorität: 12.01.2015 DE 202015000051 U
(43) Veröffentlichungstag der Anmeldung: 22.11.2017
(73) Patentinhaber: Yildiz, Fahri, 50933 Köln (DE)
(72) Erfinder: TOUSSAINT, Winfried, 64646 Heppenheim (DE)
(74) Vertreter: Kompter, Hans-Michael
(86) Internationale Anmeldenummer: PCT/DE2016/000008
(87) Internationale Veröffentlichungsnummer: WO 2016/112891

(56) Entgegenhaltungen:
- EP-A1- 1 516 604
- WO-A1-2010/025700
- US-A- 5 794 627
- US-A1- 2011 195 376
- US-A1- 2014 290 668
- US-A1- 2014 349 243

## Beschreibung

### HINTERGRUND DER ERFINDUNG

### 1. TECHNISCHES GEBIET

Die Erfindung betrifft eine vorgefertigte, zweiteilige Unterkieferprotrusionsschiene zur Behandlung von Schnarchen und/oder obstruktiver Schlafapnoe.

### 2. STAND DER TECHNIK

Schnarchen kann ein Symptom für das obstruktive Schlafapnoesyndrom sein, gekennzeichnet durch wiederholte und zahlreich auftretende nächtliche Atemstillstände, die schwerwiegende gesundheitliche Komplikationen, wie z.B. Bluthochdruck, Herz-Kreislauferkrankungen, Gehirnschlag u.a. nach sich ziehen können. Durch das US Patent US 5,462,066 sowie die europäische Patentanmeldung EP 1 203 570 sind derartige zahnspangenartige Aufbissschienen zur Verhinderung des Schnarchens bekannt geworden, welche dazu dienen, den Unterkiefer geringfügig nach vorne zu verschieben, weil in dieser Stellung des Unterkiefers die Atemwege weiter geöffnet werden, so dass der Patient freier atmen kann, ohne zu schnarchen.

Die bekannten Aufbissschienen in Form eines einteiligen, zahnspangenartigen Mundstücks bestehen aus thermoplastischen Materialien mit zwei Bissrillen, welche bei Erwärmung formbar werden. Der Patient nimmt das erwärmte, noch nicht angepasste Mundstück in den Mund, um dann in den formbaren Kunststoff die Zähne des Untersowie des Oberkiefers in die entsprechende untere sowie obere Bissrille hineinzudrücken und durch Aufbeissen auf die Bissplatten der Bissrillen anzupassen. Dabei kühlt der Kunststoff ab und gewinnt seine feste Elastizität zurück, wonach nunmehr das Mundstück an den Patienten angepasst ist. Beim Anpassungsvorgang muss darauf geachtet werden, den Unterkiefer etwas nach vorne zu verschieben, um dauerhaft einen Vorschub (Protrusion) einzustellen. Die bekannten Aufbissschienen besitzen den Nachteil, dass eine einmal eingestellte Protrusion nur schwierig sich ändernden Bedürfnissen des Patienten angepasst werden kann, so dass sich mit der Zeit der anfänglich erzielte Effekt verschlechtert.

Weiterhin schlägt das US Patent US 5,868,138 eine dentale Vorrichtung zum Behandeln von Schnarchen und obstruktivem Schlafatemstillstand vor, welches ein Oberkieferteil, ein Unterkieferteil und ein Verbindungsmittel aufweist, wobei das Verbindungsmittel das untere Teil relativ zum oberen Teil in einer vorderen vorstehenden Position einstellbar hält.

Das deutsche Gebrauchsmuster DE 29 506 512 schlägt eine oral zu tragende Antischnarchvorrichtung aus einer Ober- und Unterkieferschiene vor, wobei beide Schienen mit einem flexiblen, in Längsrichtung nicht dehnbaren Zug versehen sind, der beim Absinken des Unterkiefers diesen in eine anteriore Lage bringt.

Die Schiene aus der deutschen Patentanmeldung DE 10 2009 048 376 ist eine individuell angefertigte Schienen, die lediglich im Bereich der Backenzähne fixiert wird.

Die deutsche Patentanmeldung DE 201 02 432 schlägt eine individuell angefertigte Schiene vor, die über das komplette Gebiss fixiert wird und teleskopartige Verbinder aufweist, wobei deren Metallteile mit einer allergieverträglichen Beschichtung versehen sind.

Auch die Grundschiene von der US Patentanmeldung US 2003/0056797 Schiene wird individuell angefertigt und weist einen in diese Grundschiene eingebetteten Rahmen auf, an dem die Verbinder befestigt werden.

Die europäische Patentanmeldung EP 1 516 604 schlägt ein intraorales Therapiegerät vor bei dem die Verbinder intraokklusal angeordnet sind und der Protrusionshalter kugelgelenkig gelagert sein kann. Es findet sich keinerlei Hinweis darauf, dass die Schiene im Bereich der Frontzähne unterbrochen sein sollte.

Die Internationale Patentanmeldung WO 2006/136 684 beschreibt eine zweiteilige Vorrichtung, bei der die Unterkieferschiene die Zähne des Unterkiefers vollständig umfasst, die Oberkieferschiene aber aus 2 Teilschienen für die Backenzähne gebildet wird, die jeweils im hinteren und vorderen Bereich über einen Bogen (9, 10) miteinander verbunden sind.

Die Internationale Patentanmeldung WO 2011/017 813 schlägt ebenfalls eine individuell geformte Vorrichtung vor, bei der 4 Teilschienen an den jeweiligen Backenzähnen fixiert werden und mit einem seitlichen, elastischen Band der Vorschub erzwungen wird.

Bei den oben genannten individuell gefertigten Schienen müssen individuell im Dentallabor nach Abdrucknahme Ober- und Unterkieferschienen angefertigt werden.

Die Internationale Patentanmeldung WO 2010/025700 schlägt dagegen eine vorgefertigte, zweiteilige Unterkieferprotrusionsschiene vor, bei der sich ein thermoplastisches Füllmaterial jeweils in einer Ober- und Unterkieferschale befindet, die durch zwei starre Protrusionshalter miteinander verbunden sind. Eine entsprechende Unterkieferprotrusionsschiene mit teleskopartigen Protrusionshaltern wird in der Internationalen Patentanmeldung WO 2011/127893 beschrieben.

Solche vorgefertigten, zweiteiligen Protrusionsschienen werden von vielen Patienten in der täglichen Praxis bereits erfolgreich eingesetzt, z.B. als SomnoGuard^{®} SP. Jedoch empfinden sie manche Anwender gegenüber den im Dentallabor individuell angefertigten Protrusionsschienen immer noch als unangenehm sperrig im Gebrauch.

In der Internationalen Patentanmeldung WO 2012/038 663 wird eine der WO 2010/025700 nachempfundene Schiene vorgeschlagen, die eine geändertes Design der Protrussionsknöpfe betrifft.

Die Internationale Patentanmeldung WO 2014/016495 A1 schlägt dagegen eine ähnliche Protrusionsschiene vor, wobei jedoch die Protrusionshalter auf der hinteren Seite über einen offenen Ring an der Oberkieferschale angebracht sind.

Das US Patent US 5,794,627 schlägt eine verstellbare Protrusionsschiene mit einer unterbrochenen inneren Wand vor, bei der ein an der Oberkieferschiene angebrachter, nach unten gerichteter Pin in eines von mehreren Löchern eines an der Unterkieferschiene angebrachten, Y-förmigen Zugbandes eingreift.

Auch die inneren Wände der Oberkieferschiene des von der US Patentanmeldung US 2014/02900668 A1 vorgeschlagenen Beatmungsvorrichtung sind unterbrochen oder nicht vorhanden.

Die US Patentanmeldung US 2011/0195376 A1 schlägt Zahnschiene vor, deren Böden im Bereich der vorderen Backenzähne und Eckzähne Gelenke aufweisen.

In der US Patentanmeldung US 2014/0349243 A1 wird ein gewinkelter Protrusionshalter vorgeschlagen.

Es findet sich in den genannten Patenten und Patentanmeldungen, die vorgefertigte Protrusionsschienen betreffen, keinerlei Hinweis auf einen Schlitz im Boden der Schiene im Bereich der vorderen Schneidezähne, wobei die Schnittkanten der beiden Schienenhälften abgeschrägt sind, so dass bei einer Engerstellung der Schalenweite die beiden Schalenbodenhälften übereinander hinweg gleiten können.

Daher liegt der vorliegenden Erfindung die Aufgabe zu Grunde, solchen Patienten, die unter Schnarchen und/oder eine obstruktiven Schlafapnoe leiden und Compliance Probleme mit den bisher bekannten vorgefertigten Schienen haben, eine vorgefertigte filigranere Unterkiefer-Protrusionsschiene zur Verfügung zu stellen, welche sich bezüglich Funktionalität und Anwendungskomfort nur noch marginal von den um ein Vielfaches teureren nach Abnahme eines Zahnabdruckes durch einen Zahnarzt im Dentallabor individuell gefertigten Protrusionsschienen unterscheidet und über eine lange Zeit verwendbar bleibt.

Die Aufgabe wird durch eine Unterkieferprotrusionsschiene nach Anspruch 1 gelöst. Sie weist eine lange Lebensdauer auf und ist leicht zu handhaben. Zudem kann die erfindungsgemäße Schiene problemlos vom Patienten selbst angepasst werden, was zu einer erheblichen Erleichterung und Kostenreduktion führt. Außerdem kann der Unterkiefervorschub der erfindungsgemäßen Unterkieferprotrusionsschiene leicht an die Bedürfnisse des Patienten angepasst werden.

### KURZE ZUSAMMENFASSUNG DER ERFINDUNG

Gegenstand der Erfindung ist somit eine universell einsetzbare zweiteilige Unterkieferprotrusionsschiene zur Verhinderung von Schnarchen und/oder von obstruktiver Schlafapnoe, umfassend ein Unter- und ein Oberteil, aus einer bogenförmigen, jeweils in Gebrauch zum Unter- bzw. Oberkiefer geöffneten, Formgebenden Schale mit einem Boden, einer äußeren Wand und einer inneren Wand, wobei deren äußeren Wände an beiden Außenseiten im Bereich der Backen- bis Eckzähne einen oder mehrere Fixierungsknöpfe zur Befestigung eines Protrusionshalters aufweisen, der jeweils an einem Fixierungsknopf der Unter- und der Oberkieferschale drehbar befestigt ist und den Unterkiefer in eine posteriore oder anteriore Lage bringt, und diese Schalen jeweils ein thermoplastisches Füllmaterial enthalten, das den Zähnen des Ober- und Unterkiefers nachformbar ist, wobei die innere Wand der beiden Schalen im Bereich der Front- bis Eckzähne unterbrochen ist, und der Schalenboden mindestens einer der Schalen im Bereich der vorderen Schneidezähne mit einem Schlitz versehen ist, wobei die Schnittkanten der beiden Schienenhälften abgeschrägt sind, so dass bei einer Engerstellung der Schalenweite die beiden Schalenbodenhälften übereinander hinweg gleiten können.

Vorzugsweise ist die innere Wand der beiden Schalen im Bereich der Front- bis Eckzähne vollständig unterbrochen und im Bereich der Backenzähne auf eine minimale Wandeinfassung reduziert ist.

Durch den Einsatz mehrerer starrer Protrusionshalter unterschiedlicher Länge oder teleskopartig verstellbarer Protrussionshalter lässt sich der Unterkiefervorschub leicht den Bedürfnissen des jeweiligen Verwenders anpassen.

Die erfindungsgemäße Unterkieferprotrusionsschiene vermeidet die zeit- und kostenaufwendige individuelle Fertigung im Dentallabor nach vorheriger Abnahme eines Zahnabdruckes durch einen Zahnarzt oder Kieferorthopäden. Zudem kann diese universelle Schiene problemlos von jedem Arzt - nicht nur von Zahnärzten- oder sogar vom Patienten selbst angepasst werden können, wodurch eine erhebliche Vereinfachung in der Handhabung erzielt wird.

Ein weiterer Gegenstand der Erfindung ist ein gebrauchsfertiges Set zur Herstellung einer erfindungsgemäßen Unterkieferprotrusionsschiene zur Verhinderung von Schnarchen und/oder (obstruktiver) Schlafapnoe bestehend aus
(A) einem Unter- und einem Oberteil, aus einer bogenförmigen, jeweils in Gebrauch zum Unter- bzw. Oberkiefer geöffneten, Formgebenden Schale mit einem Boden, einer äußeren Wand und einer inneren Wand, wobei deren äußeren Wände an ihren jeweiligen Außenseiten im Bereich der Backen- bis Eckzähne einen oder mehrere Fixierungsknöpfe zur Befestigung eines starren, teleskopartig verstellbaren und/oder auszugsfähigen Protrusionshalters aufweisen, und diese Schalen jeweils ein thermoplastisches Füllmaterial enthalten, das den Zähnen des Ober- und Unterkiefers nachformbar ist, wobei die Schale im Bereich der Front- bis Eckzähne keine innere Wand aufweist, der Schalenboden mindestens einer der Schalen im Bereich der vorderen Schneidezähne mit einem Schlitz versehen ist, und die Schnittkanten der beiden Schienenhälften abgeschrägt sind, so dass bei einer Engerstellung der Schalenweite die beiden Schalenbodenhälften übereinander hinweg gleiten können,
(B) zwei oder mehrere starre, teleskopartig verstellbare und/oder auszugsfähige Protrusionshalter, und
(C) gegebenenfalls eine Gebrauchsanweisung zur Anwendung der Unterkieferprotrusionsschiene.

Die erfindungsgemäße Unterkieferprotrusionsschiene besitzt den Vorteil, dass sie vergleichbar einer dentalen Schiene sehr fest auf beiden Kiefern sitzt bzw. haftet, da aufgrund der Materialbeschaffenheit der thermoplastischen Schienenfüllung problemlos eine sehr tiefe und gleichmäßige Impression sämtlicher Zähne erreicht wird.

Des Weiteren besitzt die Unterkieferprotrusionsschiene den Vorteil, dass sie unkompliziert ohne besondere Hilfsmittel von jedem Arzt oder sogar vom Patienten selbst innerhalb weniger Minuten angepasst werden kann. Aus diesem Grund genügt auch eine universelle Standardschiene passend für fast sämtliche Kieferformationen. Des Weiteren ist in höchst vorteilhafter Weise eine individuelle Einstellung des Unterkiefervorschubs durch den Einsatz von Protrusionhaltern unterschiedlicher Länge oder von teleskopartig verstellbaren Protrusionshaltern möglich. Durch die spezielle Konstruktion der Schiene wird erreicht, dass diese sehr zierlich ist und nach Anpassung der Abstand der oberen und unteren Frontzähne mit weniger als 3 mm nur sehr klein ist, was sich sehr positiv auf Tragekomfort und Akzeptanz auswirkt und auch darauf, dass die Schiene gleichzeitig für unterschiedlich große Kieferformationen geeignet ist.

### KURZE BESCHREIBUNG DER ABBILDUNGEN

Figur 1 zeigt eine perspektivische Ansicht von schräg unten und vorne einer Ausführungsform der erfindungsgemäßen Protrusionsschiene;
Figur 2 zeigt eine perspektivische Ansicht von der Seite der gleichen Ausführungsform der erfindungsgemäßen Protrusionsschiene;
Figur 3 zeigt eine Ansicht von vorne der gleichen Ausführungsform der erfindungsgemäßen Protrusionsschiene;
Die Figur 4 und 5 zeigen schematische Darstellungen einer Ausführungsform der ungefüllten Oberkieferschale;
Figur 6 zeigt eine schematische Seitenansicht einer teleskopartig verstellbaren Ausführungsform des erfindungsgemäßen Protrusionshalters;
Figur 7 zeigt eine schematische Darstellung in der Aufsicht dieses Protrusionshalters;
Figur 8 zeigt eine Seitenansicht einer ausziehbaren und teleskopartig verstellbaren Ausführungsform des erfindungsgemäßen Protrusionshalters;
Figur 9 zeigt eine schematische Darstellung in der Aufsicht dieses Protrusionshalters in ausgezogener (A) und zusammengeschobener Form (B).

### DETAILLIERTE BESCHREIBUNG DER ERFINDUNG

Die erfindungsgemäße Protrusionsschiene lässt sich aus bekannten Materialien universell massengefertigt zum Beispiel im Spritzgussverfahren herstellen.

Geeignete, starre Protrusionshalter werden zum Beispiel in der WO 2010/025700 beschrieben. Die teleskopartig und gegebenenfalls ausziehbaren Protrusionshalter können hergestellt werden, indem man die jeweiligen mit Befestigungselementen versehenen Teleskophülsen im Spritzgussverfahren herstellt und mit einem Verbindungselement miteinander verbindet.

Vorzugsweise werden die starren Protrusionshalter und die mit Befestigungselementen versehenen Teleskophülsen aus Polyoxymethylen (POM) gefertigt. Zur Herstellung der Verbindungselemente eignet sich Edelstahl, insbesondere V4A Edelstahl.

Der Begriff "Protrusionsschiene" bzw. "Unterkieferprotrusionsschiene" wie er vor- und nachstehend verwendet wird bezeichnet eine dentale Vorrichtung, welche es erlaubt, den Unterkiefer gegenüber dem Oberkiefer in eine leicht nach vorne gelagerte Position zu verschieben und dadurch den Querschnitt der oberen Atemwege zu vergrößern. Dies führt zu einer Reduktion des Schnarchens und von Atemaussetzern infolge einer obstruktiven Schlafapnoe.

Der Begriff "vorgefertigt" wie er vor- und nachstehend in Bezug auf die erfindungsgemäße Unterkieferprotrusionsschiene verwendet wird bezeichnet eine Unterkieferprotrusionsschiene, die im Gegensatz zu den individuell, durch Abformen des Kiefers des Anwenders durch den Zahnarzt hergestellten Schienen, im industriellem Maßstab unabhängig von den individuellen Größen oder Formen der Kiefer der späteren Anwender hergestellt wird. Die jeweilige spätere Anpassung an die Kieferform bzw. -größe erfolgt durch Einbiss des Anwenders in das erwärmte und dadurch weiche thermoplastische Material und anschließendes Abkühlen und Erhärten dieses Materials.

Der Begriff "thermoplastisches Füllmaterial" wie er vor- und nachstehend verwendet wird bezeichnet ein Material, das sich in der Wärme, vorzugsweise unterhalb von 70 °C, vorzugsweise zwischen 40 und 65 °C plastisch verformen lässt und sich eng an eine vorgegebene Form anschmiegt und danach beim Abkühlen diese besagte Form beibehält. Geeignete Materialien sind u.a. z.B. Polymere und Kopolymere oder Gemische davon aus der Gruppe der Polyethylene (PE), Polyvinylacetate (PVA), Acrylate und Methacrylate, bevorzugt Kopolymere aus PE und PVA, wie sie zum Beispiel unter der Marke Elvax^{®} der Firma DuPont erhältlich sind. Bevorzugt sind solche PE-PVA Kopolymere, deren PVA Gehalt von 20 bis 40, insbesondere 25 bis 35 % beträgt, sowie Polycaprolacton.

Besonders bevorzugt ist Polycaprolacton, das nach Anpassung der Schalen beim Abkühlungsprozess aushärtet und die Zähne in einer dünnen festen Schicht umschließt. Entsprechende Polycaprolactone sind zum Beispiel unter dem Handelsnamen Pearlbond^{®} von der Firma Lubrizol Advanced Materials Manufacturing Spain, S.L., Gran Vial no. 17, Montmelo 08160 Barcelona oder CAPA^{®} 6500 oder CAPA^{®} 6800 von Perstorp UK Limited, Baronet Rd., Warrington Wa4 6HA, UK, erhältlich.

Da Polycaprolacton beim Abkühlen um etwa ein Prozent schrumpft und fest ausgekühlt, wird zudem eine vorzügliche Retention (Zahnhaftung) erreicht, welche der Retention von zahnärztlich gefertigten individuellen Unterkieferprotrusionsschienen nicht mehr nachsteht.

Der Begriff "Formgebende Schale" wie er vor- und nachstehend verwendet wird bezeichnet einen "U-, bogen- bzw. hufeisenförmigen" Formkörper, der eine an den Enden offene Schale oder Wanne ausbildet. In der Regel besteht dieser Formkörper aus einem unter physiologischen Bedingungen inerten und stabilen Material wie zum Beispiel duroplastischen Kunststoffen, wie zum Beispiel Polytetrafluoroethylen (PTFE, Teflon^{®}), oder Polykarbonaten oder aus Elastomeren wie zum Beispiel Polyacrylaten.

Der Begriff "Fixierungsknopf" wie er vor- und nachstehend verwendet wird bezeichnet eine stabförmige Erhebung mit einer endständigen Verdickung, die jeweils an der Seite der Unter- bzw. Oberkieferschale angebracht sind, und an denen der Protrusionshalter reversibel befestigt werden kann. In der Regel ist der Fixierungsknopf kugelkopfförmig ausgestaltet, so dass die endständige Verdickung in eine Aussparung des Protrusionshalters, welche vorzugsweise die Form einer Kugelgelenkpfanne aufweist, einrastet, diesen somit um die Längsachse des Fixierungsknopfes drehbar fixiert und gleichzeitig auf Grund der bevorzugten Kugelgelenkfunktion eine leichte, seitliche Bewegung der Kiefer gegeneinander nicht behindert.

Der Begriff "Protrusionshalter" wie er vor- und nachstehend verwendet wird bezeichnet ein Verbindungselement zwischen der Ober- und der Unterkieferschale, welches es ermöglicht, dass der Unterkiefer im getragenen Zustand in eine anteriore Lage gebracht wird. Er weist endständige Aussparungen auf, in die jeweils ein Fixierungsknopf , vorzugsweise in Form einer Gelenkpfanne einrasten kann. Die Geometrie des Protrusionshalters an sich ist unkritisch; er sollte lang genug sein, um den Unterkiefer in eine anteriore Lage zu bringen. Die starre Ausführungsform des Protrusionshalters sollte gegenüber Dehnungen oder Stauchungen stabil sein. Dagegen sollte ein teleskopartig verstellbarer Protrusionshalter einen leicht bedienbaren Mechanismus zur Verstellung der Länge aufweisen und nach erfolgter Einstellung ebenso gegenüber Dehnungen oder Stauchungen stabil sein. Er muss relativ dünn sein, um einen guten Tragekomfort zu gewährleisten. In einer alternativen Ausführungsform kann ein teleskopartig verstellbarer Protrusionshalter auch so ausgeführt sein, dass er auf einer Seite auszugsfähig ist, wodurch er leichten Öffnungen des Mundes bis zu einem gewissen, vorgegebenen Grad nachgibt, was zu einem weiterhin erhöhten Tragekomfort führt.

Die vor- und nachstehend verwendeten Angaben hinsichtlich der Geometrie oder räumlichen Anordnung der Protrusionsschiene oder von Teilen davon orientieren sich an den Gegebenheiten der Protrusionsschiene bei ihrer sinngemäßen Verwendung, d.h. nach Einsetzen derselben auf die obere und untere Zahnreihe der betroffenen Person: "in Längsrichtung" bedeutet in Richtung der Mundöffnung ("anterior") oder des Rachens ("posterior"); "nach vorne" (anterior) bedeutet in Richtung der Mundöffnung; "mittig" bedeutet im Bereich der Schneidezähne; "hinten" bedeutet im Bereich der hinteren Backenzähne; "seitlich" bedeutet im Bereich zwischen den Prämolaren und den hintern Molaren.

Die Erfindung ist im beigefügten Anspruchssatz beschrieben. Vorteilhafte Ausgestaltungen der Erfindung sind solche erfindungsgemäßen Unterkieferprotrusionsschienen, wobei
(a) die äußere Wand im Bereich im Bereich zwischen den Eck-und Backenzähnen niedriger oder unterbrochen ist; vorzugsweise weist die in diesem Bereich verbleibende Außenwand eine Höhe von weniger als 2,0 mm, insbesondere 1,2 bis 1,8 mm, besonders bevorzugt etwa 1,5 mm auf;
(b) die äußere Wand im Bereich der Frontzähne und der Fixierungsknöpfe eine Gesamthöhe von 4,0 bis 6,0 mm, vorzugsweise von 4,5 bis 5,5 mm, insbesondere etwa 4,75 mm aufweist;
(c) die Fixierungsknöpfe einen Durchmesser von 3,5 bis 6,0 mm, vorzugsweise von 3,8 bis 5,2 mm insbesondere etwa 4,4 mm aufweisen
(d) die äußeren Wände der Ober- und Unterkieferschalen an beiden Außenseiten im Bereich der Backenzähne und im Bereich der Eckzähne, vorzugsweise in einem Abstand von 25,0 bis 35,0 mm, insbesondere von 27,0 bis 31,0 mm, besonders bevorzugt von etwa 28,7 mm gemessen zwischen jeweils zwei Fixierungsknöpfen, bzw. von etwa 29,8 mm gemessen von der jeweiligen Mitte der Fixierungsknöpfe, jeweils einen Fixierungsknopf zur Befestigung eines Protrusionshalters aufweisen.
(e) das Füllmaterial (4, 5) ein biokompatibles, toxikologisch unbedenkliches Polymer oder Co-polymer ist, das sich in der Wärme, vorzugsweise unterhalb von 70 °C, insbesondere zwischen 40 und 65 °C plastisch verformen lässt und sich eng an eine vorgegebene Form anschmiegt und danach beim Abkühlen diese besagte Form beibehält, vorzugsweise besteht es im Wesentlichen aus einem oder mehreren Copolymeren aus Polyethylen und Polyvinylacetat oder aus Polycaprolacton. Insbesondere weist das Füllmaterial einen Einbisskanal auf;
(f) die Formgebende Schale im Wesentlichen aus Polycarbonat besteht;
(g) die Länge des Protrusionshalters über eine in der Mitte befindliche Mutter mit zwei gegenläufigen Gewinden teleskopartig verstellbar ist und jeweils an beiden Enden eine ringförmige Aussparung zur Aufnahme der Fixierungsknöpfe aufweist;
(h) das eine Ende des Protrusionshalters mit der ringförmigen Aussparung gegenüber dem Rest des Protrusionhalters abgewinkelt ist;
(i) der Protrusionshalter über einen Auszug verlängert werden kann;
(j) die Fixierungsknöpfe als Kugelgelenksköpfe ausgebildet sind und die jeweiligen Protrusionshalter über eine Kugelgelenkspfanne drehbar daran befestigt sind; dabei beträgt der Durchmesser des Kugelgelenkskopfes in seiner Mitte vorzugsweise 4,0 bis 4,5 mm, insbesondere 4,2 bis 4,4 mm, ganz besonders bevorzugt etwa 4,3 mm und an den beiden äußeren Rändern jeweils 3,0 bis 3,5 mm, insbesondere 3,2 bis 3,4 mm, ganz besonders bevorzugt etwa 3,3 mm.

Weiterhin bevorzugt sind im Falle der starren Protrusionshalter solche, die aus einem elastischen und reißfesten Material, insbesondere aus Polyoxymethylen (POM, z.B. Ultraform^{®} / BASF) oder im Falle der teleskopartig verstellbaren Protrusionshalter solche, deren mit den Befestigungselementen versehenen Teleskophülsen ebenfalls aus einem elastischen und reißfesten Material, insbesondere aus POM hergestellt sind und über ein Verbindungselement aus Edelstahl miteinander verbunden sind.

Der Abstand zwischen der Innenseite des Bodens der Oberkieferschale und der Innenseite des Bodens der Unterkieferschale, im zusammengesetzten Zustand beträgt vorzugsweise weniger als 8 mm, insbesondere 1 bis 5 mm, besonders bevorzugt etwa 2 mm; dies führt dazu dass der der Abstand der Zähne bei angelegter Schiene im Frontzahnbereich bei etwa 2 bis 4 mm liegt. Besonders bevorzugt ist ein Abstand der Zähne im Frontzahnbereich von etwa 2 bis 3 mm bei angelegter Schiene.

Dadurch kann der Mund auch bei kleinen Kiefergrößen ohne Muskelanspannung leicht und dabei vollständig geschlossen werden. Dadurch resultieren höherer Tragekomfort und damit auch eine bessere Akzeptanz (Compliance / Adhärenz).

Das verwendete Polymer oder Co-Polymer des thermoplastischen Füllmaterials kann im Spritzgussverfahren separat hergestellt werden, und nach leichtem Erwärmen seiner Oberfläche auf die Innenseiten der beiden ungefüllten Schalen leicht angepresst werden. Nach Erkalten sind die beiden Komponenten fest miteinander verbunden. Die polymere Masse haftet absolut fest auf der Kontaktfläche aus Polycarbonat.

Sollte weiterhin nach langer Tragezeit über viele Monate die Haftung der Schiene an Zähnen und Kiefern nachlassen, kann man so ebenfalls problemlos durch Erwärmen und Einbeißen die Anpassung erneut vornehmen. Die erfindungsgemäße Unterkieferprotrusionsschiene kann somit gegebenenfalls nach zwischenzeitlichen Wiederherstellungen über einen sehr langen Zeitraum uneingeschränkt verwendet werden.

Ausführungsbeispiele der Erfindung sind in den Zeichnungen dargestellt und werden im Folgenden näher beschrieben.

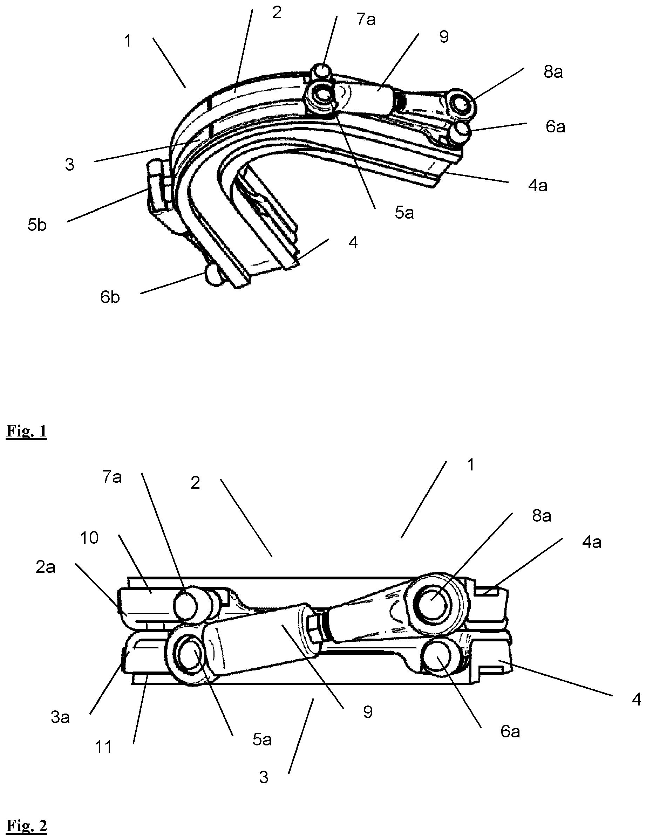

Figur 1 zeigt eine perspektivische Ansicht von schräg unten und vorne der erfindungsgemäßen Protrusionsschiene (1), wobei der erfindungsgemäße Schlitz durch das Füllmaterial verdeckt ist, gebildet aus einem Oberteil (2) und einem Unterteil (3), die jeweils aus einer Schale und einem Füllmaterial (4) gebildet werden. Dieses Füllmaterial weist einen Einbisskanal (4a) auf. Dieser erleichtert das korrekte Einbeißen beim Anpassen der Schiene. Der Einbisskanal hat in der Regel eine Breite von 7,3 bis 8,5 mm, vorzugsweise 7,6 bis 8,2 mm, insbesondere etwa 7,8 mm im Backenzahnbereich auf. Im Frontzahnbereich ist der Einbisskanal um etwa 40 %, d.h. auf insbesondere etwa 5,6 mm verengt. Weiterhin weisen sowohl das Oberteil (2) als auch das Unterteil (3) der Protrusionsschiene (1) jeweils auf beiden Seitenbereichen zwei Fixierungsköpfe (5a, 6a, 7a, 8a, 5b, 6b, 7b, 8b) auf, wobei die beiden vorderen Fixierungsknöpfe des Unterteils (5a, 5b) jeweils über einen Protrusionshalter (9) mit den beiden hinteren Fixierungsknöpfen (8a, 8b) des Oberteils verbunden sind.

In einer bevorzugten Ausführungsform weisen die Ober- (2a) und Unterschale (3a) (vgl. Fig. 2) eine identische Form auf. Zur ihrer Herstellung werden jeweils etwa 2,0 bis 4,0 cm³ Polycarbonat verwendet.

Zur Herstellung der Füllung der Unter- und Oberschale werden jeweils etwa 5,0 bis 10,0 cm³ Polycaprolacton eingesetzt.

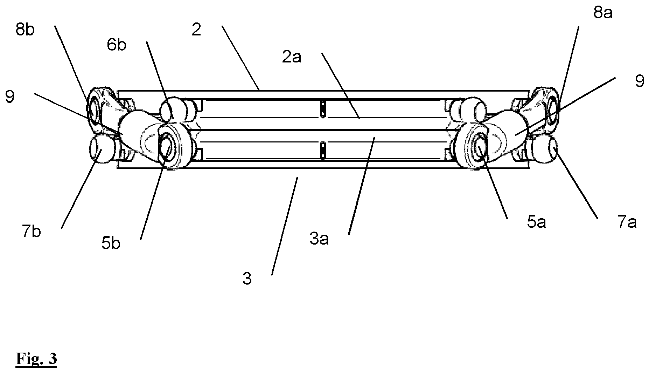

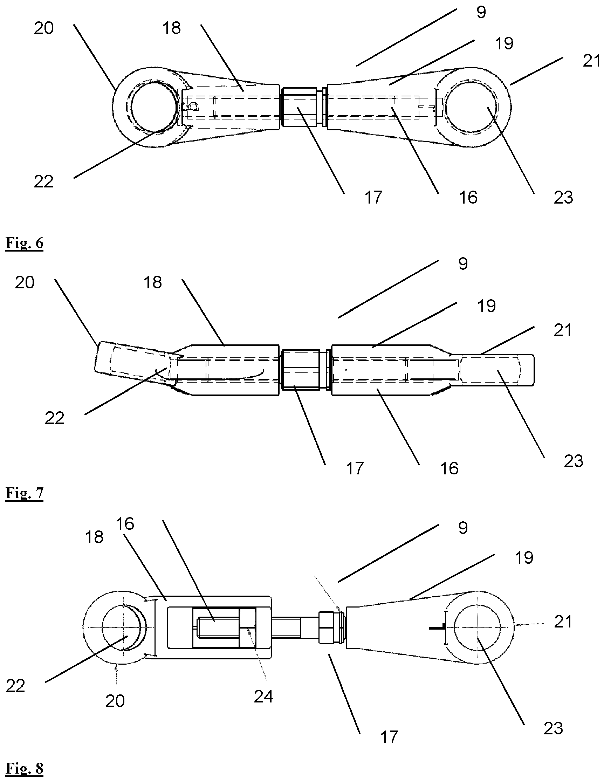

Die Figuren 2 und 3 zeigen perspektivische Ansichten der gleichen Ausführungsform wie Fig. 1 jeweils von der Seite (Fig. 2) und von vorne (Fig. 3. Diese Protrusionsschiene wird gebildet aus einem Oberteil (2) und einem Unterteil (3), die jeweils aus einer Schale (2a, 3a) und einem Füllmaterial (4) gebildet werden. Dieses Füllmaterial weist einen Einbisskanal (4a) auf. Weiterhin weisen sowohl das Oberteil (2) als auch das Unterteil (3) der Protrusionsschiene (1) von Fig. 2 auf den gezeigten Seite zwei Fixierungsköpfe (5a, 6a, 7a, 8a) auf, wobei der vordere Fixierungsknopf des Unterteils (5a) jeweils über einen Protrusionshalter (9) mit dem hinteren Fixierungsknopf (8a) des Oberteils verbunden ist. Dieser dargestellte Protrusionshalter ist sowohl auszugfähig als auch teleskopartig verstellbar (vgl. Fig. 7 und Fig. 8).

Wie aus Fig. 2 weiterhin zu erkennen ist, überragt das thermoplastische Material (4) jeweils die Höhe der Außenwände (10, 11) der Formgebenden Schalen (2a, 3a), vorzugsweise im Bereich der Schneidezähne und hinteren Backenzähne um 0,5 bis 2,0 mm, insbesondere um 1 bis 1,5 mm und im Bereich der vorderen Backenzähne um 1,5 bis 3,5 mm, insbesondere um 2,0 bis 3,0 mm. Dies führt zu einer verbesserten Einfassung der Zähne.

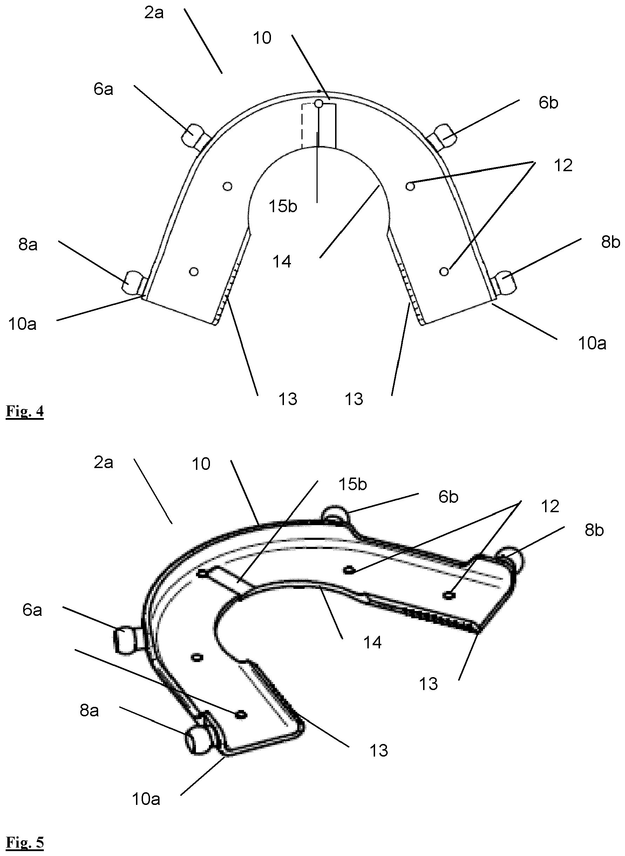

In den Figures 4 und 5 ist die Protrusionsschiene mit dem erfindungsgemäßen Schlitz (15b) im Schalenboden im Bereich der vorderen Schneidezähne abgebildet. Dabei ist der Schalenboden vorzugsweise zu mehr als 50 % seiner Breite mit einem Schlitz versehen. Die Länge des Schlitzes beträgt insbesondere 6,0 bis 9,0 mm, besonders bevorzugt etwa 8 mm.

Die Schnittkanten der beiden Schienenhälften sind abgeschrägt, so so dass bei einer Engerstellung der Schalenweite die beiden Schalenbodenhälften übereinander hinweg gleiten können. Wird die Schalenweite vergrößert, wird sich der Schlitz geringfügig verbreitern. Durch das Übereinanderhinweggleiten der Schalenbodenhälften wird vermieden, dass bei der Anpassung das plastische Füllmaterial in nennenswertem Ausmaß aus dem Schalenbogen austritt.

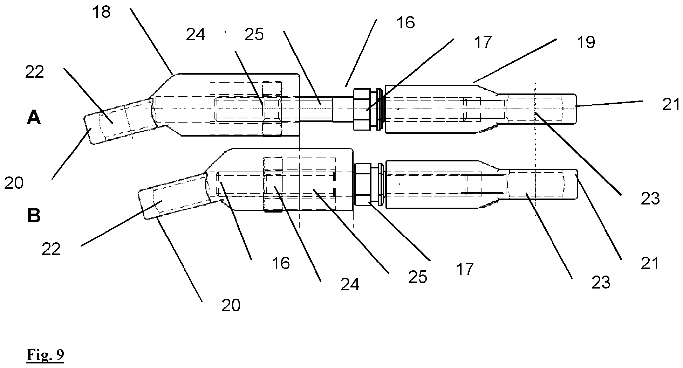

Figur 6 zeigt eine schematische Seitenansicht einer teleskopartig verstellbaren, aber nicht auszugsfähigen Ausführungsform des erfindungsgemäßen Protrusionshalters (9) mit den beiden an den jeweiligen, seitlich vollständig geschlossenen Teleskophülsen (18, 19) angebrachten endständigen Befestigungselementen (20, 21) und deren ringförmigen Aussparungen (22, 23). Das Verbindungselement (16) ist als eine Sechskantspindel mit gegenläufigem Gewinde ausgestaltet und kann über das Sechskant-Profil (17) auf Grund der in den Teleskophülsen befindlichen Innengewinde mit Hilfe eines filigranen Sechskantschlüssels verstellt werden. Der Durchmesser des Sechskantprofils beträgt vorzugsweise etwa 3 mm, der Durchmesser des in die Kunststoffhülse eingebrachten Gewindes beträgt in der beispielhaften Ausführungsform der Fig. 9 etwa 2,9 mm.

Das Verbindungselement (16) weist vorzugsweise im nicht prolongierten Zustand eine Gesamtlänge von 18 bis 25 mm, insbesondere 20 bis 22 mm, ganz besonders bevorzugt von etwa 21 mm. Vorzugsweise ist es aus einem austenitischen Edelstahl gefertigt, insbesondere der Klasse A2-50 oder A4-50(70).

Die beiden Teleskophülsen (18, 19) mit den endständigen angebrachten Befestigungselementen (20, 21) sind beispielsweise aus POM hergestellt und weisen vorzugsweise eine Länge von 9,0 bis 12,5 mm, insbesondere 10,0 bis 11,5 mm ganz besonders bevorzugt etwa 10,9 mm gemessen vom Beginn des Innengewindes bis zum Mittelpunkt der jeweiligen ringförmigen Aussparung (22, 23) auf. Die Innenseiten der ringförmigen Aussparungen (22, 23) sind nach innen gewölbt. In ihrer Mitte weisen sie einen Innendurchmesser von vorzugsweise 4,6 bis 5,0 mm, insbesondere 4,7 bis 4,9 mm, ganz besonders bevorzugt etwa 4,8 mm auf, und an ihren beiden Rändern haben sie jeweils einen Innendurchmesser von vorzugsweise 4,2 bis 4,6 mm, insbesondere 4,3 bis 4,5 mm, ganz besonders bevorzugt etwa 4,4 mm auf, und bilden somit Kugelgelenkspfannen zur Aufnahme der nach außen gewölbten Fixierungsköpfe aus.

Mit diesem teleskopartig verstellbaren Protrusionshalter lässt sich der Vorschub per Schub wie zum Beispiel in Fig. 1 gezeigt erzielen, kann aber auch per Zug erfolgen, wenn man diesen Protrusionhalter an den Fixierungsknöpfen (6a) und (7a) sowie (6b) und (7b) befestigt (vgl. Fig. 3).

Figur 7 zeigt eine schematische Darstellung in der Aufsicht dieses Protrusionshalters, dessen vorderes Befestigungselement (20) gegenüber der entsprechenden Teleskophülse (18) leicht abgewinkelt ist. Der zwischen (20) und (18) gebildete Winkel ist kleiner als 180° und liegt vorzugsweise bei 150 bis 178°, insbesondere bei 160 bis 175°.

Figur 8 zeigt eine Seitenansicht einer ausziehbaren und teleskopartig verstellbaren Ausführungsform des erfindungsgemäßen Protrusionshalters. Während die hintere Teleskophülse (19) und die beiden Befestigungselemente (20, 21) ebenso ausgestaltet sind wie die des Protrusionshalters der Fig. 6, ist die vordere Teleskophülse (18) einseitig offen. Diese Teleskophülse weist im Gegensatz zur Teleskophülse (19) kein Innengewinde auf, sondern umschließt den Außenumfang der Stellschraube (24), wodurch sich das Verbindungselement (16) zwischen dem Anschlag der Stellschraube (24) an die von dem Befestigungselement (20) abgewandte Seite der Teleskophülse (18) und dem Anschlag der Sechskantmutter (17) ausziehen lässt. Die maximale Länge dieses Auszugs (25) kann durch Betätigen der Sechskant-Mutter (17) entsprechend eingestellt werden. Der Auszug (25) kann dementsprechend mit Hilfe der Stellschraube (17) zwischen 0,0 und 10 mm, vorzugsweise zwischen 8,0 und 2,0 mm, insbesondere zwischen 4,0 und 5,0 mm frei eingestellt werden. Der Auszug fährt zum Beispiel bei anliegender Schiene bei Mundöffnung aus, wobei die eingestellte Protrusion des Unterkiefers fortbestehen bleibt.

Figur 9 zeigt eine schematische Darstellung in der Aufsicht dieses Protrusionshalters in ausgezogener (A) mit einem Auszug von etwa 4,5 mm und zusammengeschobener Form (B), dessen vorderes Befestigungselement (20) gegenüber der entsprechenden Teleskophülse (18) leicht abgewinkelt ist. Der zwischen (20) und (18) gebildete Winkel ist kleiner als 180° und liegt vorzugsweise bei 150 bis 178°, insbesondere bei 160 bis 175°, beispielweise bei etwa 170°. Die Innenseiten der ringförmigen Aussparungen (22, 23) sind nach innen gewölbt und bilden dadurch Kugelpfannen zur Aufnahme der nach außen gewölbten Fixierungsköpfe.

Der Abstand der beiden Wände (11,13) der Schale an der posterioren Seite beträgt vorzugsweise 14,0 bis 16,0 mm, insbesondere 14,2 bis 15,6 mm ganz besonders bevorzugt etwa 14,8 mm.

Die Wandstärke der Außenwände (10, 11,), der Innenwände (13) und der jeweiligen Böden beträgt vorzugsweise 1,0 bis 2,0 mm, insbesondere 1,2 bis 1,8 mm, besonders bevorzugt etwa 1,5 mm.

Das Anpassen der erfindungsgemäßen Protrusionsschiene an den Kiefer der betroffenen Person ist einfach. Die Anpassung kann im Regelfall von jedem Arzt gleich welcher Fachrichtung, dessen instruiertem Personal oder zumeist auch von der betroffenen Person alleine mit Hilfe eines Spiegels durchgeführt werden.

Bei Vorliegen von Zahnfehlstellungen, anamnestischen Kiefergelenksbeschwerden oder Zahnerkrankungen wie z.B. Parodontose u.a. ist es ratsam, vor Anpassung einen Zahnarzt zu konsultieren.

Bei der Anpassung der erfindungsgemäßen Schiene erwärmt man zuerst das Oberteil (2) auf eine Temperatur oberhalb der Erweichungstemperatur des Füllmaterials (4), vorzugsweise in einem Wasserbad bei einer Wassertemperatur von oberhalb 50 °C, insbesondere in einem kochenden Wasserbad etwa für 20-30 Sekunden, wobei das Füllmaterial plastisch wird. Danach passt man das Oberteil an die Zahnreihe des Oberkiefers an, indem man die Zähne in das weiche, plastische und noch warme Füllmaterial gleichmäßig tief eindrückt. Anschließend lässt man das Füllmaterial kurz etwa für 30 - 60 Sekunden im Mund abkühlen und dann endgültig in einem kalten Wasserbad aushärten.

Danach erwärmt man das Unterteil (3) entsprechend und passt es wie oben beschrieben an die Zähne des Unterkiefers an. Danach werden die beiden angepassten Kieferschalen mithilfe von 2 Protrusionshaltern gleicher Länge verbunden. Hinsichtlich der auszugsfähigen Teleskopverbinder (vgl. Figs. 8, 9) wird empfohlen, diese stets im Backenzahnbereich der Oberkieferschale (vgl. Fig. 3: 8a, 8b)und im Frontbereich der Unterkieferschale zu fixieren (vgl. Fig. 3: 5a, 5b), d.h. die Protrusion des Unterkiefers wird per Schub realisiert. Lediglich bei Verwendung der nicht auszugsfähigen Teleskope (vgl. Figs. 9, 10) kann die Vorschubeinstellung in Abhängigkeit von der damit realisierbaren Unterkieferprotrusion auch durch die gegenläufige Anbringung der Teleskopverbinder erfolgen, also vorne oben (Fig. 3: 6a, 6b) und hinten unten (Fig. 3: 7a, 7b).

Die erfindungsgemäße Unterkieferprotrusionsschiene beseitigt oder reduziert Schnarchen und Atemaussetzer infolge einer obstruktiven Schlafapnoe. Durch Ihre extrem zierliche Größe weist sie einen sehr hohen Tragekomfort auf. Sie vermittelt einen festen, sicheren Halt für die umfassten Zähne, kann mit Hilfe der teleskopartig verstellbaren Protrusionshalter stufenlos hinsichtlich des Unterkiefervorschubs an die Bedürfnisse des Patienten angepasst werden, und schützt die Zähne gegen nächtliches Zähneknirschen.

## Patentansprüche

1. Zweiteilige Unterkieferprotrusionsschiene (1) zur Verhinderung von Schnarchen und/oder von obstruktiver Schlafapnoe, umfassend ein Unter- (2) und ein Oberteil (3), aus einer bogenförmigen, jeweils in Gebrauch zum Unter- bzw. Oberkiefer geöffneten, Formgebenden Schale mit einem Boden, einer äußeren Wand (10) und einer inneren Wand (13), wobei deren äußeren Wände an beiden Außenseiten im Bereich der Backen- bis Eckzähne einen oder mehrere Fixierungsknöpfe (5a, 6a, 7a, 8a, 5b, 6b, 7b, 8b) zur Befestigung eines Protrusionshalters (9) aufweisen, der jeweils an einem Fixierungsknopf der Unter- und der Oberkieferschale drehbar befestigt ist und den Unterkiefer in eine posteriore oder anteriore Lage bringt, und diese Schalen jeweils ein thermoplastisches Füllmaterial (4) enthalten, das den Zähnen des Ober- und Unterkiefers nachformbar ist, wobei die innere Wand mindestens einer der Schalen im Bereich der Front- bis Eckzähne unterbrochen ist, und der Schalenboden mindestens einer der Schalen im Bereich der vorderen Schneidezähne mit einem Schlitz (15b) versehen ist, der die Schale in zwei Schienenhälften mit je einer Schalenbodenhälfte unterteilt, **dadurch gekennzeichnet, dass** die Schnittkanten der beiden Schienenhälften abgeschrägt sind, so dass bei einer Engerstellung der Schalenweite die beiden Schalenbodenhälften übereinander hinweg gleiten können.

2. Unterkieferprotrusionsschiene nach Anspruch 1, **dadurch gekennzeichnet, dass** der Schalenboden zu mehr als 50 % seiner Breite mit dem Schlitz (15b) versehen ist.

3. Unterkieferprotrusionsschiene nach Anspruch 1 oder 2, **dadurch gekennzeichnet, dass** die äußere Wand (10) mindestens einer der Schalen im Bereich zwischen den Eck-und Backenzähnen unterbrochen ist.

4. Unterkieferprotrusionsschiene nach einem der Ansprüche 1 bis 3, **dadurch gekennzeichnet, dass** die äußere Wand (10) mindestens einer der Schalen im Bereich der Frontzähne und der Fixierungsknöpfe eine Gesamthöhe von 4,0 bis 6,0 mm, vorzugsweise von 4,5 bis 5,5 mm, insbesondere etwa 4,75 mm aufweist.

5. Unterkieferprotrusionsschiene nach einem der Ansprüche 1 bis 4, **dadurch gekennzeichnet, dass** die äußeren Wände (10) der Ober- und Unterkieferschalen an beiden Außenseiten im Bereich der Backenzähne und im Bereich der Eckzähne jeweils einen Fixierungsknopf zur Befestigung eines Protrusionshalters (9) aufweisen.

6. Unterkieferprotrusionsschiene nach einem der Ansprüche 1 bis 5, **dadurch gekennzeichnet, dass** der Boden beider Schalen im Bereich der Front- bis Eckzähne mit dem Schlitz (15b) versehen ist.

7. Unterkieferprotrusionsschiene nach einem der Ansprüche 1 bis 6, **dadurch gekennzeichnet, dass** die Schichtdicke des thermoplastischen Füllmaterial (4) 2 bis 4 mm, vorzugsweise etwa 3 mm beträgt.

8. Unterkieferprotrusionsschiene nach einem der Ansprüche 1 bis 7, **dadurch gekennzeichnet, dass** das Füllmaterial (4) ein biokompatibles, toxikologisch unbedenkliches Polymer oder Kopolymer ist, das sich in der Wärme, vorzugsweise unterhalb von 70 °C, insbesondere zwischen 40 und 65 °C plastisch verformen lässt und sich eng an eine vorgegebene Form anschmiegt und danach beim Abkühlen diese besagte Form beibehält.

9. Unterkieferprotrusionsschiene nach einem der Ansprüche 1 bis 8, **dadurch gekennzeichnet, dass** die Länge des Protrusionshalters (9) über eine in der Mitte befindliche Mutter (17) mit zwei gegenläufigen Gewinden teleskopartig verstellbar ist und jeweils an beiden Enden eine ringförmige Aussparung (22) zur Aufnahme der Fixierungsknöpfe aufweist.

10. Unterkieferprotrusionsschiene nach einem der Ansprüche 1 bis 9, **dadurch gekennzeichnet, dass** das eine Ende des Protrusionshalters (9) mit der ringförmigen Aussparung (22) gegenüber dem Rest des Protrussionhalters abgewinkelt ist.

11. Unterkieferprotrusionsschiene nach einem der Ansprüche 1 bis 10, **dadurch gekennzeichnet, dass** der Protrusionshalter (9) über einen Auszug (25) verlängert werden kann.

12. Unterkieferprotrusionsschiene nach einem der Ansprüche 1 bis 11, **dadurch gekennzeichnet, dass** die Fixierungsknöpfe als Kugelgelenksköpfe ausgebildet sind und die jeweiligen Protrusionshalter (9) über eine Kugelgelenkspfanne drehbar daran befestigt sind.

13. Gebrauchsfertiges Set zur Herstellung einer Unterkieferprotrusionsschiene zur Verhinderung von Schnarchen und/oder obstruktiver Schlafapnoe nach einem der Ansprüche 1 bis 12 bestehend aus
(A) einem Unter- (2) und einem Oberteil (3), aus einer bogenförmigen, jeweils in Gebrauch zum Unter- bzw. Oberkiefer geöffneten, Formgebenden Schale mit einem Boden, einer äußeren Wand (10) und einer inneren Wand (13), wobei deren äußeren Wände an ihren jeweiligen Außenseiten im Bereich der Backen- bis Eckzähne einen oder mehrere Fixierungsknöpfe (5a, 6a, 7a, 8a, 5b, 6b, 7b, 8b) zur Befestigung eines starren, teleskopartig verstellbaren und/oder auszugfähigen Protrusionshalters (9) aufweisen, und diese Schalen jeweils ein thermoplastisches Füllmaterial (4) enthalten, das den Zähnen des Ober- und Unterkiefers nachformbar ist, wobei mindestens eine der Schalen im Bereich der Front- bis Eckzähne keine innere Wand aufweist, der Schalenboden mindestens einer der Schalen im Bereich der vorderen Schneidezähne mit einem Schlitz (15b) versehen ist, der die Schale in zwei Schienenhälften mit je einer Schalenbodenhälfte unterteilt, und die Schnittkanten der beiden Schienenhälften abgeschrägt sind, so dass bei einer Engerstellung der Schalenweite die beiden Schalenbodenhälften übereinander hinweg gleiten können,
(B) zwei oder mehrere starre, teleskopartig verstellbare und/oder auszugsfähige Protrusionshalter (9), und
(C) gegebenenfalls eine Gebrauchsanweisung zur Anwendung der Unterkieferprotrusionsschiene.

14. Gebrauchsfertiges Set nach Anspruch 13, **dadurch gekennzeichnet, dass** das Unter- (2) und das Oberteil (3) (A) im Wesentlichen identisch sind.

## Claims

1. A two-part mandibular advancement splint (1) for preventing snoring and/or obstructive sleep apnoea, comprising a lower (2) and an upper part (3) made of an arcuate moulding tray, in each case open to the lower or upper jaw when in use, with a base, an exterior wall (10), and an interior wall (13), wherein the outer walls thereof have one or more fixation knobs (5a, 6a, 7a, 8a, 5b, 6b, 7b, 8b) on both outer sides in the area from the molars to the canines for fastening an advancement holder (9), which is fastened rotatably respectively to one fixation knob each of the lower and upper tray and brings the mandible into a posterior or anterior position, and each of these trays contains a thermoplastic filling material (4) that can be shaped to conform to the teeth of the maxilla and mandible, wherein the interior wall of at least one of the trays is interrupted in the area of the front teeth to the canines, and the tray base of at least one of the trays is provided with a slit (15b) in the area of the anterior incisors separating the tray into two splint halves, each with one tray base half, **characterized in that** the cutting edges of the two splint halves are tapered so that, when the width of the trays is narrowed, the two tray base halves can slide over one another.

2. The mandibular advancement splint according to claim 1, **characterized in that** more than 50% of the width of the tray base is provided with said slit (15b).

3. The mandibular advancement splint according to claim 1 or 2, **characterized in that** the exterior wall (10) of at least one of the trays is interrupted in the area between the canines and molars.

4. The mandibular advancement splint according to any of claims 1 to 3, **characterized in that** the exterior wall (10) of at least one of the trays has an overall height of 4.0 to 6.0 mm, preferably from 4.5 to 5.5 mm, in particular about 4.75 mm, in the area of the front teeth and the fixation knobs.

5. The mandibular advancement splint according to any of claims 1 to 4, **characterized in that** the exterior walls (10) of the upper and lower trays each have a fixation knob for fastening an advancement holder (9) on both outer sides in the area of the molars and in the area of the canines.

6. The mandibular advancement splint according to any of claims 1 to 5, **characterized in that** the base of both trays is provided with the slit (15b) in the area of the front teeth to the canines.

7. The mandibular advancement splint according to any of claims 1 to 6, **characterized in that** the layer thickness of the thermoplastic filling material (4) is 2 to 4 mm, preferably about 3 mm.

8. The mandibular advancement splint according to any of claims 1 to 7, **characterized in that** the filling material (4) is a biocompatible, toxicologically safe polymer or copolymer, which can undergo plastic deformation under heating, preferably at below 70°C, in particular between 40 and 65°C, and adapts closely to a given shape and then retains said shape upon cooling.

9. The mandibular advancement splint according to any of claims 1 to 8, **characterized in that** the length of the advancement holder (9) is telescopically adjustable via a central nut (17) with two opposing threads and an annular recess (22) at both ends for receiving the fixation knobs.

10. The mandibular advancement splint according to any of claims 1 to 9, **characterized in that** one end of the advancement holder (9) with the annular recess (22) is angled relative to the rest of the advancement holder.

11. The mandibular advancement splint according to any of claims 1 to 10, **characterized in that** the advancement holder (9) can be extended via an extension (25).

12. The mandibular advancement splint according to any of claims 1 to 11, **characterized in that** the fixation knobs are designed as ball joint heads and the respective advancement holders (9) are rotatably attached thereto via a ball joint socket.

13. A ready-to-use kit for making a mandibular advancement splint to prevent snoring and/or obstructive sleep apnoea according to any of claims 1 to 12, consisting of
(A) a lower (2) and an upper part (3) made of an arcuate moulding tray, in each case open to the lower or upper jaw when in use, with a base, an exterior wall (10), and an interior wall (13), wherein the outer walls thereof have one or more fixation knobs (5a, 6a, 7a, 8a, 5b, 6b, 7b, 8b) on the respective outer sides thereof in the area of the molars to the canines for attaching a rigid, telescopically adjustable and/or extendable advancement holder (9), and each of these trays contains a thermoplastic filling material (4) that can be shaped to conform to the teeth of the maxilla and mandible, wherein at least one of the trays has no interior wall in the area of the front teeth to the canines, the tray base of at least one of the trays is provided with a slit (15b) in the area of the front incisors, which slit divides the tray into two splint halves, each with a tray base half, and the cutting edges of the two splint halves are tapered so that, when the width of the tray is narrowed, the two tray base halves can slide over one another;
(B) two or more rigid, telescopically adjustable and/or extendable advancement holders (9); and
(C) instructions for use of the mandibular advancement splint, if applicable.

14. The ready-to-use kit according to claim 13, **characterized in that** the lower part (2) and the upper part (3) (A) are essentially identical.

## Revendications

1. Gouttière de propulsion mandibulaire en deux parties (1) pour éviter le ronflement et/ou l'apnée obstructive du sommeil, comprenant une partie inférieure (2) et une partie supérieure (3) constituées d'une coque de moulage en forme d'arc, chacune ouverte sur le maxillaire inférieur ou supérieur lors de l'utilisation, avec un fond, une paroi externe (10) et une paroi interne (13), dont les parois externes comportent un ou plusieurs boutons de fixation (5a, 6a, 7a, 8a, 5b, 6b, 7b, 8b) des deux côtés extérieurs dans la zone des molaires jusqu'aux canines pour la fixation d'un support de propulsion (9), qui est fixé de manière rotative à un bouton de fixation des coques de maxillaires inférieur et supérieur et place le maxillaire inférieur dans une position postérieure ou antérieure, et ces coques contiennent chacune un matériau de remplissage thermoplastique (4) qui peut être modelé sur les dents des maxillaires supérieur et inférieur, la paroi interne d'au moins l'une des coques étant interrompue dans la zone des dents antérieures jusqu'aux canines, et le fond de coque d'au moins l'une des coques dans la zone des incisives antérieures est pourvue d'une fente (15b) dont la coque est divisée en deux moitiés de gouttière, chacune avec une moitié de fond de coque, **caractérisée en ce que** les arêtes de coupe des deux moitiés de gouttière sont biseautées, de sorte que lorsque la largeur de coque est réduite, les deux moitiés de fond de coque peuvent glisser l'une sur l'autre.

2. Gouttière de propulsion mandibulaire selon la revendication 1, **caractérisée en ce que** le fond de la coque est pourvue de la fente (15b) sur plus de 50% de sa largeur.

3. Gouttière de propulsion mandibulaire selon la revendication 1 ou 2, **caractérisée en ce que** la paroi externe (10) d'au moins une des coques est interrompue dans la zone comprise entre les canines et les molaires.

4. Gouttière de propulsion mandibulaire selon l'une des revendications 1 à 3, **caractérisée en ce que** la paroi externe (10) d'au moins une des coques au niveau des dents antérieures et des boutons de fixation a une hauteur totale de 4,0 à 6,0 mm, de préférence de 4,5 à 5,5 mm, en particulier d'environ 4,75 mm.

5. Gouttière de propulsion mandibulaire selon l'une des revendications 1 à 4, **caractérisée en ce que** les parois externes (10) des coques du maxillaire supérieur et inférieur comportent chacune un bouton de fixation pour fixer un support de propulsion (9) sur les deux côtés externes dans la zone des molaires et dans la zone des canines.

6. Gouttière de propulsion mandibulaire selon l'une des revendications 1 à 5, **caractérisée en ce que** le fond des deux coques est pourvue de la fente (15b) dans la région des dents antérieures jusqu'aux canines.

7. Gouttière de propulsion mandibulaire selon l'une des revendications 1 à 6, **caractérisée en ce que** l'épaisseur de couche du matériau de remplissage thermoplastique (4) est de 2 à 4 mm, de préférence d'environ 3 mm.

8. Gouttière de propulsion mandibulaire selon l'une des revendications 1 à 7, **caractérisée en ce que** le matériau de remplissage (4) est un polymère ou copolymère biocompatible, toxicologiquement inoffensif qui peut être déformé plastiquement à chaud, de préférence en dessous de 70°C, en particulier entre 40 et 65°C et s'adapte étroitement à une forme donnée, puis conserve cette dite forme lorsqu'il refroidit.

9. Gouttière de propulsion mandibulaire selon l'une des revendications 1 à 8, **caractérisée en ce que** la longueur du support de propulsion (9) est réglable télescopiquement par l'intermédiaire d'un écrou (17) situé en son milieu à deux filets opposés et comporte un évidement annulaire (22) aux deux extrémités destinées à recevoir les boutons de fixation.

10. Gouttière de propulsion mandibulaire selon l'une des revendications 1 à 9, **caractérisée en ce qu'**une extrémité du support de propulsion (9) avec l'évidement annulaire (22) est coudée par rapport au reste du support de propulsion.

11. Gouttière de propulsion mandibulaire selon l'une des revendications 1 à 10, **caractérisée en ce que** le support de propulsion (9) peut être prolongé par une extension (25).

12. Gouttière de propulsion mandibulaire selon l'une des revendications 1 à 11, **caractérisée en ce que** les boutons de fixation sont conçus comme des têtes d'articulation sphériques et le support de propulsion respectif (9) y est fixé de manière rotative via une cuvette de rotule.

13. Kit prêt à l'emploi pour fabriquer une gouttière de propulsion mandibulaire pour éviter le ronflement et/ou l'apnée obstructive du sommeil selon l'une des revendications 1 à 12 consistant en
(A) une partie inférieure (2) et une partie supérieure (3), constituées d'une coque de moulage en forme d'arc chacune ouverte sur le maxillaire inférieur ou supérieur lors de l'utilisation, avec un fond, une paroi externe (10) et une paroi interne (13), dont les parois externes comportent un ou plusieurs boutons de fixation (5a, 6a, 7a, 8a, 5b, 6b, 7b, 8b) sur leurs faces extérieures respectives dans la zone des molaires jusqu'aux canines pour la fixation d'un support de propulsion (9) rigide, réglable télescopiquement et/ou extensible, et ces coques contiennent chacune un matériau de remplissage thermoplastique (4) qui peut être modelé sur les dents des maxillaires supérieur et inférieur, au moins une des coques n'ayant pas de paroi interne dans la zone des dents antérieures jusqu'aux canines, le fond d'au moins une des coques est pourvue d'une fente (15b) dans la zone des incisives antérieures, qui divise la coque en deux moitiés de gouttière, chacune avec une moitié de fond de coque, et les bords coupés des deux moitiés de gouttière sont biseautés, de sorte que lorsque la largeur de coque est réduite, les deux moitiés de fond de coque peuvent glisser l'une sur l'autre en se chevauchant,
(B) deux support(s) de propulsion(s) (9) rigide(s), réglable(s) télescopiquement et/ou extensible(s) ou plus, et
(C) une notice d'utilisation de la gouttière de propulsion mandibulaire, le cas échéant.

14. Kit prêt à l'emploi selon la revendication 13, **caractérisé en ce que** la partie inférieure (2) et supérieure (3) (A) sont sensiblement identiques.
